# EUROPEAN PATENT APPLICATION

(11) **EP 4 312 468 A1**
(43) Date of publication of application: **31.01.2024**
(21) Application number: 22187713.7
(22) Date of filing: 29.07.2022
(51) Int. Cl.: H05G 1/02, A61B 6/00

(54) **COOLING SYSTEM FOR AN X-RAY DEVICE**

(71) Applicant: Koninklijke Philips N.V., 5656 AG Eindhoven (NL)
(72) Inventor: RAMACHANDRAIAH, Mallikarjun, Eindhoven (NL); DE BOER, Jacob, Eindhoven (NL)
(74) Representative: Philips Intellectual Property & Standards

(57) **Abstract**

The invention provides a cooling system for an X-ray device, the cooling system comprising a top cooling unit, TCU, comprising a heat exchanger plate with an integrated cooling fluid circuitry, and a support arm, particularly a C-arc, configured for mounting the TCU and for mounting a monoblock 16 of an X-ray source 17 of the X-ray device, wherein the fluid circuitry is arranged over a main surface of the heat exchanger plate so as to capture heat via the main surface.

## Description

### FIELD OF THE INVENTION

The invention provides a cooling system for an X-ray device, and an X-ray device.

### BACKGROUND OF THE INVENTION

X-ray devices as presently used, particularly mobile X-ray systems, often comprise an X-ray source comprising an X-ray tank, also referred to as a monoblock, which comprises an X-ray tube and may also comprise a high voltage generator integrated into one physical assembly. The monoblock comprises a tank with a cooling fluid which is pumped inside the monoblock to circulate the cooling fluid internally to distribute the heat inside the monoblock. In other words, at least part of the cooling system is integrated with the X-ray source, particularly the monoblock.

The present systems suffer from several challenges, including difficulty in assembling and servicing the systems or exchanging the X-ray source and difficulty in keeping the performance of the cooling circuit up over an extended period of time.

It is an objective of the present invention to provide a cooling system for an X-ray device, and an X-ray device that alleviate at least some of the above challenges.

### SUMMARY OF THE INVENTION

The object is achieved by the present invention. The invention provides a cooling system and an X-ray device according to the independent claims. Embodiments are laid down in the dependent claims.

The invention provides a cooling system for an X-ray device, the cooling system comprising a top cooling unit, TCU, comprising a heat exchanger plate with an integrated cooling fluid circuitry, and a support arm, particularly a C-arc, configured for detachably mounting the TCU and for detachably mounting a monoblock of an X-ray source of the X-ray device, wherein the fluid circuitry is arranged over a main surface of the heat exchanger plate so as to capture heat via the main surface.

Thus, a cooling system may be provided that allows for reliably retracting heat from the monoblock mounted on the support arm, e.g., from the top surface of the monoblock. At the same time, as the cooling system is not incorporated in the monoblock, it is possible to exchange the monoblock/X-ray source without disconnecting the cooling circuit, thereby keeping the performance of the cooling circuit in good condition. When the cooling circuit is interrupted, i.e., it cannot stay complete or connected, this may lead to decreased performance of the cooling circuit and decreased reliability.

The cooling system allows for an easy initial assembly and easy mounting and dismounting of parts independently of each other. Improved servicing may thus also be achieved by the configuration of the cooling system. The cooling system allows for modular construction and for being used as an add-on cooling system for existing X-ray devices.

The cooling system also allows for increasing maximum operation time/exposure time/procedure time of the X-ray device, as it provides very efficient cooling, i.e., removing heat from the monoblock, thereby preventing overheating for an extended time.

The cooling system is particularly advantageous for use in mobile X-ray devices and/or X-ray devices with many back-to-back procedures.

In the present disclosure, unless otherwise specified, all relative arrangements of elements refer to an assembled state of the cooling system and/or the X-ray device for intended use.

A plate, according to the present disclosure, may have two oppositely arranged surfaces, e.g., top and bottom surfaces, connected by one or more side surfaces. Particularly, a height of the plate, may be smaller than the length and width of the top and bottom surfaces and/or smaller than a radius of the top and bottom surfaces. In particular, the side surfaces may each be smaller than the top and bottom surfaces.

The main surface of the heat exchanger plate of the present disclosure may be one of the larger surfaces, in particular in an assembled state, the surface facing the monoblock. In an assembled state, the main surface may be in contact with a surface of the monoblock.

The fluid circuitry being arranged over the main surface may entail that the fluid circuitry is arranged in a plane that is parallel to the plane of the main surface, particularly in plane that is at least as close to the main surface as it is to the surface opposite the main surface.

Exemplary configurations of a heat exchanger plate the fluid circuitry being arranged over a main surface of the heat exchanger plate so as to capture heat via the main surface will be presented in detail below. It is to be understand that these are mere examples of possible configurations. As an example, the feature "so as to capture heat via the main surface" may refer to being configured such that, when the cooling system is put to its intended use, i.e. with a cooling fluid inside of, particularly circulating through, the fluid circuitry, heat is captured via the main surface, e.g., from an object, like a monoblock, arranged adjacent to the main surface. Thus, the skilled person will be aware that various arrangements and shapes of the fluid circuitry will allow for capturing heat via the main surface.

In operation, cooling fluid circulating through the heat exchanger plate may thus capture heat from the monoblock.

The cooling fluid circuitry being integrated in the heat exchanger plate may entail that the cooling fluid channels of the cooling fluid circuitry are formed inside the heat exchanger plate, particularly formed integrally with the heat exchanger plate.

The heat exchanger plate may be made from one piece or assembled from multiple pieces, particularly, multiple pieces joined together permanently. If the heat exchanger plate is made from multiple pieces, the cooling fluid circuitry may be formed by or in one or more of said multiple pieces.

The cooling fluid may be water, oil, and/or glycol.

The support arm may be seen as having a tank-holder portion, which comprises a mounting mechanism for the TCU and for the monoblock. The support arm may further, at an end opposite the end than where the TCU is mounted, have a portion configured for mounting an X-ray detector of the X-ray device.

The support arm, particularly the C-arc, may be made as an extrusion profile, particularly initially in a straight shape. In order to obtain a curved shaped, it may then be rolled.

The cooling system may be configured such that, in operation, cooling fluid may be made to flow through the TCU to transfer heat away from the X-ray source, for example, to transfer heat to the free environment via a heat exchanger, e.g., an external heat exchanger fluidly connected to the TCU and/or an additional heat exchanger, like a water or air heat exchanger.

The support arm may be configured such that it may fully support the TCU or, in other words, such that the TCU can be held in a suspended position by the support arm alone.

According to the present disclosure, the integrated cooling fluid circuitry may comprise at least one, in particular two or more, integrated cooling fluid channels. Each of the cooling fluid channels may have two ports, in particular, an inlet port and an outlet port. The ports are also referred to herein as ports of the TCU or as ports of the heat exchanger plate.

Each cooling fluid channel may extend in a plane perpendicular to the main surface of the heat exchanger plate. In particular, all cooling fluid channels may extend in the same plane.

Alternatively or in addition, each cooling fluid channel may be loop-shaped, the loop starting at one port and ending at the other port. In case there are two or more cooling fluid channels, at least two cooling fluid channels may be loop-shaped and one of the loops may be arranged entirely inside of the other loops.

Each of the ports may be integrally formed with the heat exchanger plate or attached to the heat exchanger plate.

The two ports of the cooling fluid channel, in particular all ports of all of the cooling fluid channels may be arranged at the same side of the heat exchanger plate, in particular, extend from the same side surface of the heat exchanger plate.

Alternatively or in addition the two ports of the cooling fluid channel, in particular, all the ports of all of the cooling fluid channels may be arranged in parallel to each other. Being arranged in parallel to each other may, in particular, refer to the main flow direction of fluid within the ports.

The configuration of the fluid channels as described herein allows for high heat exchange efficiency and small dimensions of the heat exchanger plate. Moreover, it may allow for easy connecting and disconnecting of the heat exchanger plate, particularly the cooling fluid circuitry, with an external fluid circuitry, e.g., an external heat exchanger circuitry. As an example, parallel arrangement and/or arrangement on the same side and/or arrangement in the same plane may allow for easy mounting, e.g., by a plug connection.

According to the present disclosure, the TCU may comprise an interface section configured to engage with an interface section of the support arm. In particular, the interface section of the TCU and the interface section of the support arm may be configured to provide an assembly mechanism configured such that the TCU is detachably fixed to the support arm in an assembled state.

The interface section of the TCU may comprise the ports of the one or more integrated cooling fluid channels. For example, each of the ports may serve as one of a pair of connection elements of a plug-type connection. Alternatively or in addition, the interface section of the TCU may comprise one or more other elements that serve as one of a pair of connection elements of a plug-type connection. The respective other one of the pair of connection elements of a plug-type connection may be comprised in the interface section of the support arm. Thus, the assembly mechanism may comprise a plug connection.

Alternatively or in addition, the interface section of the TCU may comprise one or more holes and/or grooves, each configured to receive a pin of the interface section of the support arm, and/or may comprise one or more pins configured to be received in a hole and/or groove of the interface section of the support arm. The assembly may be achieved by engaging each of the pins with its respective hole or groove. This allows for aligning the TCU and/or fixing the relative position of the TCU and the support arm and/or supporting the TCU. Thus, the assembly mechanism may comprise a position fixing mechanism.

Alternatively or in addition, the assembly mechanism may comprise a sliding mechanism and the interface section of the TCU may comprise one of a pair of elements constituting the sliding mechanism, for example, a slot or groove configured to guide a matching portion of the interface section of the support arm, or a portion that is configured as a matching portion for a slot or a groove of the interface section of the support arm.

Thus, an assembly mechanism may be provided by the interface section of the TCU and the interface section of the support arm.

It is noted that a groove of the interface section of the TCU may serve as both, a part of a fixing mechanism and a part of a sliding mechanism of the assembly mechanism.

The above-described assembly mechanisms may also provide support for the TCU on the support arm, i.e., comprise a support mechanism, which may comprise at least a subset of the elements of the position fixing mechanism and/or of the sliding mechanism, for example.

According to the present disclosure, the support arm may comprise a mounting mechanism for detachably mounting the monoblock to the support arm, in particular in such a manner that mounting and dismounting of the monoblock does not require detaching the TCU from the support arm.

This is particularly advantageous for exchanging the monoblock over the lifetime of an X-ray device easily and without compromising the cooling circuit.

The mounting mechanism of the support arm and the assembly mechanism, in particular, the interface section of the support arm, may be configured such that, in an assembled state, the main surface of the heat exchanger plate is arranged over and in contact with the top surface of the monoblock.

The mounting mechanism for detachably mounting the monoblock to the support arm may optionally be comprised in the interface section of the support arm. This may allow for a compact configuration of the cooling system and/or X-ray device.

The mounting mechanism of the support arm may comprise a screwing mechanism and/or a click-mechanism and/or a clamping mechanism.

As an example, the mounting mechanism of the support arm may be configured such that the support arm can be attached to the monoblock when the TCU and the support arm are in an assembled state. For example, the mounting mechanism may be such that the TCU and support arm, in an assembled state, can be placed on top of the monoblock and the monoblock can then be fixed to the support arm, e.g., by means of screws or a click-mechanism or a clamping mechanism. As an example, a plurality of holes for receiving screws may extend from top to bottom of the interface section of the support arm, such that the monoblock can be fixed to the support arm by placing the support arm on top of the monoblock, inserting a screw from the top into each the plurality of holes, and screwing the monoblock to the support arm.

The mounting mechanism of the support arm may be configured such that the weight of the monoblock may be fully held or supported by the support arm.

According to the present disclosure, the cooling system, in particular the TCU, may comprise a mounting mechanism for detachably attaching the TCU to the monoblock, in particular the top surface of the monoblock, mounted on the support arm.

This is particularly advantageous for exchanging the monoblock over the lifetime of an X-ray device easily and without compromising the cooling circuit.

The mounting mechanism for detachably attaching the TCU to the monoblock may, in particular, be configured such that main surface of the heat exchanger plate is held in contact with the top surface of the monoblock in an assembled state.

The mounting mechanism for detachably attaching the TCU to the monoblock may optionally at least partially be arranged outside of the interface section of the TCU. In particular, it may be arranged closer to a second end than to a first end of the heat exchanger plate where the interface section of the TCU is arranged, the first and second ends being opposite ends of the heat exchanger plate.

For example, in the area of the interface section of the TCU, the relative position of the TCU and the monoblock will be mainly determined by the support arm's mounting mechanism and the assembly mechanism. Accordingly, it is particularly advantageous to ensure contact of the main surface with the monoblock in regions removed from the interface section of the TCU.

The mounting mechanism for detachably attaching the TCU to the monoblock may comprise a screwing mechanism and/or a click-mechanism and/or a clamping mechanism.

As an example, the mounting mechanism for detachably attaching the TCU to the monoblock may be configured such that the TCU can be attached to the monoblock when the TCU and the support arm are in an assembled state and optionally when the monoblock is mounted on the support arm. For example, the mounting mechanism may be configured such that the TCU placed on top of the monoblock, e.g. after assembling the TCU and the support arm and after mounting the monoblock to the support arm, can be attached to the monoblock, e.g., by means of screws or a click-mechanism or a clamping mechanism. As an example, a plurality of holes for receiving screws may extend from top to bottom of the TCU, particularly the heat exchanger plate, such that the TCU can be attached to the monoblock by placing the TCU on top of the monoblock, inserting a screw from the top into each the plurality of holes, and screwing the TCU to the support arm.

According to the present disclosure, the TCU may comprise a mounting mechanism for mounting a collimator of the X-ray device. In particular, the heat exchanger plate may have an opening in its main surface for accommodating at least a portion of the collimator and optionally may comprise positioning elements, e.g., grooves and/or holes and/or pins, for positioning the collimator on top of the heat exchanger plate. The opening may, in particular, extend from the main surface of the heat exchanger plate to the opposite surface of the heat exchanger plate, i.e., be a through-hole extending through the heat exchanger plate. The mounting mechanism may serve to align the collimator with respect to the monoblock as well.

The opening may be configured and the collimator in a mounted state may be arranged and configured such that X-rays emitted from the X-ray source are able to travel through the opening and into the collimator. In particular, the opening may be configured such that a portion of the collimator is arranged in the opening and another portion of the collimator is supported by a portion of the surface opposite the main surface of the heat exchanger plate.

The positioning elements may be configured to engage with corresponding positioning elements of the collimator, for example grooves and/or holes and/or pins. In particular, the TCU may comprise two or more pins extending orthogonally from the surface opposite of the main surface of the heat exchanger plate. The collimator may have corresponding holes into which the pins may be inserted for positioning/aligning the collimator, e.g., by sliding it onto the TCU.

The mounting mechanism for mounting the collimator may optionally comprise a depression and/or recess in the top surface of the TCU, particularly the heat exchanger plate, i.e., the surface opposite to the main surface or the surface facing away from the monoblock in an assembled state. The depression and/or recess may be configured to allow for accommodating the collimator, in particular so as to limit a sideways movement of the collimator with respect to the top surface of the TCU, while supporting the collimator from the bottom. This may serve for providing additional stability. Optionally, the mounting mechanism for mounting the collimator may also comprise an attachment mechanism, for example a screwing mechanism and/or a clamping mechanism, for fixing the collimator to the TCU.

A mounting mechanism for mounting the collimator according to the present disclosure allows for easy assembly and disassembly and may also allow for a compact configuration of the X-ray device.

The collimator may be provided to avoid beam leakage and execute beam shaping.

According to the present disclosure, the support arm, in particular the C-arc, may comprise one or more cooling channels, each having two ports connected, in an assembled state, to the two ports of a cooling fluid channel of the cooling fluid circuitry, such that, in an assembled state, one or more closed cooling circuits are formed, each by at least one of the cooling channels of the support arm and at least one of the cooling fluid channels of the heat exchanger plate.

The support arm, in particular the cooling channels of the support arm, may be configured so as to allow for heat to be exchanged between fluid circulating through the cooling channels and the surroundings of the support arm. The support arm offers enough room for a cooling fluid to travel a long way, thereby allowing for efficient heat exchange.

The support arm may, thus, be configured to radiate heat. The support arm may serve as a heat exchanger that is external and connected to the TCU. It is noted that, alternatively, the TCU may also be connected to a heat exchanger that is separate from the support arm. The separate heat exchanger may, optionally, be supported by the support arm.

In particular, the support arm, e.g., C-arc, may comprise a first cooling channel extending along a first support arm/C-arc side and arranged to have two open ends to be connected to two ports of the heat exchanger plate, e.g., two of the ports of the cooling fluid channels. Furthermore, the support arm, e.g., C-arc, may comprise a second cooling channel extending along a second support arm/C-arc side, preferably opposite to the first support arm/C-arc side, and arranged to have two open ends to be connected to two other ports of the heat exchanger plate, e.g., two of the ports of the cooling fluid channels. The open ends of the first cooling channel and/or the second cooling channel may serve as ports of the cooling channels.

The TCU may be mounted on the support arm and the support arm may be coupled with the TCU in such a manner that the coupling allows for cooling fluid from the TCU to be circulated through the cooling channels of the support arm, in particular, so as to allow for closed loop circulation through the support arm and the TCU. In other words, the cooling channels of the support arm and the cooling fluid channels of the TCU may form one or more cooling circuits. The cooling channels in the support arm may thus be part of the complete cooling system together with the TCU.

The cooling system may additionally comprise an air ventilator in the support arm, e.g., for additional cooling. For example, the support arm may comprise air conduits through which cooling air may pass to cool the support arm. This may provide an additional cooling boost.

According to the present disclosure, the heat exchanger plate and the support arm, in particular their respective interface sections, may be configured such that the ports of the support arm and the ports of the one or more cooling fluid channels are configured so as to form a plug connection with each other.

For example, the plug connection may allow for connecting the support arm and the TCU, particularly for attaching the TCU and/or for establishing the one or more closed cooling circuits, by sliding the TCU towards the support arm so as to engage the ports of heat exchanger plate and the corresponding ports of the support arm.

This allows for easy and reliable assembly.

According to the present disclosure, the cooling system may comprise the/an assembly mechanism configured such that the TCU is detachably fixed to the support arm and the assembly mechanism may comprise the plug connection and fixing elements, for example pins, configured to lock the relative position of the TCU and the support arm when the plug connection is in a connected state. As an example, the assembly mechanism described above may be comprised in the cooling system. The plug connection may, in particular, be a plug connection formed by the ports of the heat exchanger plate and the ports of the support arm, for example as described above. An easy and reliable assembly can be ensured by such an assembly mechanism.

According to the present disclosure, the cooling system may further comprise one or more pumps for circulating cooling fluid through the cooling fluid circuitry of the TCU and, in particular, through the closed cooling circuits formed by the cooling channels of the support arm and the cooling fluid channels of the heat exchanger plate.

The one or more pumps may comprise any suitable pump known in the art. The one or more pumps may, for example, be arranged at the end of the support arm opposite the end having the interface section, e.g., the end where the X-ray detector is attached. This is advantageous in terms of the overall distribution of constituting elements of the cooling system or X-ray device.

According to the present disclosure, the one or more integrated cooling fluid channels may be arranged so as to optimize heat distribution throughout the heat exchanger plate.

As an example, the one or more integrated cooling fluid channels may be loop-shaped, as described above. As an example, a first loop-shaped cooling fluid channel may extend along the outer sidewalls of the heat exchanger plate. As another example, a second loop-shaped cooling fluid channel may extend around the inner sidewalls of the heat exchanger plate, e.g., along the edges of any openings in the heat exchanger plate, like the opening for accommodating a portion of the collimator.

Thus, efficient heat exchange can be achieved.

In an optional embodiment, the TCU is arranged for mounting a collimator of the X-ray device such that the heat exchange plate is located between the monoblock and the collimator. The integrated cooling fluid circuitry of the heat exchange plate comprises a portion of channel extending beneath the collimator once mounted and above the monoblock, or in other words between the collimator and the monoblock. In a particular embodiment, the heat exchanger plate has an opening in its main surface for accommodating at least a portion of the collimator and a shape of said portion of channel closely follows an external shape of this opening - this could be said second loop-shaped cooling fluid channel extending around the inner sidewalls of the heat exchanger plate, e.g., along the edges of this opening or any openings in the heat exchanger plate.

Again, efficient heat exchange can be achieved.

According to the present disclosure, the cooling channels of the support arm may extend along the entire length of the support arm. For example, in case of a C-arc, they may extend along the whole arc.

In particular, the two ports of each cooling channel, i.e., an input port and an output port, may be arranged at the same end of the support arm and each of the cooling channels may extend from said end of the support arm to the other end of the support arm and back.

The above may improve utilization of available space, e.g., in the support arm, for heat exchange.

The present invention also provides an X-ray device comprising the cooling system of the present disclosure, e.g., according to the claims or as described above, and an X-ray source comprising a monoblock, in particular, wherein, in an assembled state, the main surface of the heat exchanger plate is in contact with the top surface of the monoblock.

According to the present disclosure, the X-ray device may further comprise an X-ray detector, e.g., arranged at the end of the support arm opposite to the end of the support arm on which the monoblock and TCU are mounted. The pumps mentioned above may be arranged at the same end of the support arm as the X-ray detector.

Another embodiment of the invention comprises a cooling system for an X-ray device, the cooling system comprising
a top cooling unit, TCU, comprising a heat exchanger plate with an integrated cooling fluid circuitry; and
a support arm, particularly a C-arc, configured for mounting the TCU and for mounting a monoblock of an X-ray source of the X-ray device.

The fluid circuitry is arranged over a main surface of the heat exchanger plate so as to capture heat via the main surface. The TCU is further arranged for mounting a collimator of the X-ray device such that the heat exchange plate is located between the monoblock and the collimator. The integrated cooling fluid circuitry of the heat exchange plate comprises a portion of channel extending beneath the collimator once mounted and above the monoblock, or in other words between the collimator and the monoblock. In a particular embodiment, the heat exchanger plate has an opening in its main surface for accommodating at least a portion of the collimator and a shape of said portion of channel closely follows, or extends along, an external shape of this opening - this could be a loop-shaped cooling fluid channel extending around the inner sidewalls of the heat exchanger plate, e.g., along the edges of this opening or any openings in the heat exchanger plate.

More efficient heat exchange can be achieved.

As another embodiment, the invention comprises a top cooling unit, TCU, comprising a heat exchanger plate with an integrated cooling fluid circuitry arranged for mounting with a support arm, particularly a C-arc, mounted with a monoblock of an X-ray source of an X-ray device. The fluid circuitry is arranged over a main surface of the heat exchanger plate so as to capture heat via the main surface. Optionally, TCU is particularly arranged for being detachably mounted to a support arm. As another alternative or combined option, the TCU is further arranged for mounting a collimator of the X-ray device such that the heat exchange plate is located between the monoblock and the collimator, wherein the integrated cooling fluid circuitry of the heat exchange plate comprises a portion of channel extending beneath the collimator once mounted and above the monoblock, or in other words between the collimator and the monoblock, wherein in a particular embodiment the heat exchanger plate has an opening in its main surface for accommodating at least a portion of the collimator and a shape of said portion of channel closely follows, or extends along, an external shape of this opening - this could be a loop-shaped cooling fluid channel extending around the inner sidewalls of the heat exchanger plate, e.g., along the edges of this opening or any openings in the heat exchanger plate. The features and advantages outlined above in the context of the cooling system similarly apply to the X-ray device described herein.

Further features, examples, and advantages will become apparent from the detailed description making reference to the accompanying drawings.

### BRIEF DESCRIPTION OF THE DRAWINGS

Figs. 1a and 1b illustrate schematically a TCU of a cooling system according to the present disclosure;
Figs. 2a and 2b illustrate schematically an assembly mechanism for detachably fixing a TCU according to the present disclosure;
Fig. 3 illustrates schematically a TCU and a portion of the support arm of a cooling system according to the present disclosure and a collimator;
Fig. 4 illustrates schematically a TCU and a portion of the support arm of a cooling system according to the present disclosure and a monoblock;
Fig. 5 illustrates an exemplary support arm of the present disclosure; and
Figs. 6a to 6c illustrate an X-ray device according to the present disclosure.

### DETAILED DESCRIPTION OF EMBODIMENTS

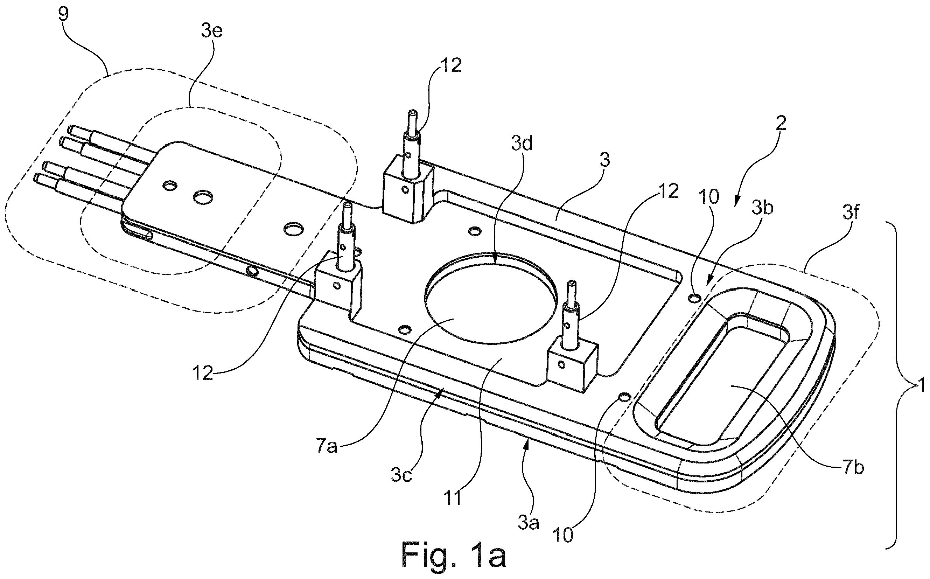

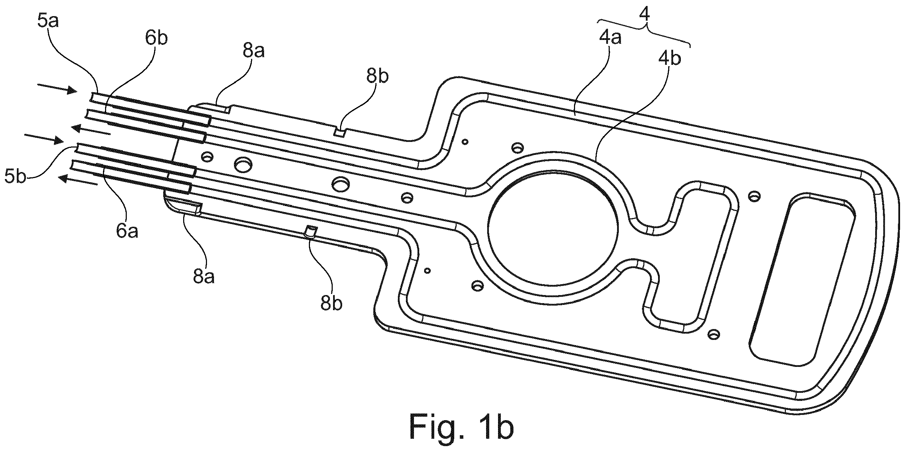

Figs. 1a and 1b illustrate schematically a TCU 2 of a cooling system 1 according to the present disclosure in an oblique view from the top and in a cross-sectional view.

The TCU comprises a heat exchanger plate 3 with an integrated cooling fluid circuitry 4 comprising two cooling fluid channels 4a, 4b, which, as shown in the figures, may be loop-shaped. Alternative shapes are conceivable. There may also be a different number of cooling fluid channels.

The TCU also comprises, at a first end 3e of the heat exchanger plate, four ports, i.e., an inlet port 5a and an outlet port 6a of the cooling fluid channel 4a and an inlet port 5b and an outlet port 6b of the cooling fluid channel 4b. The ports may be formed integrally with the heat exchanger plate and accordingly with the cooling fluid channels, or they may be attached to the heat exchanger plate. In any case, they are referred to, for the sake of simplicity, as ports of the cooling fluid channels. The ports may serve for fluidly connecting the TCU to an external heat exchanger. A second end 3f of the heat exchanger plate is arranged oppositely to the first end.

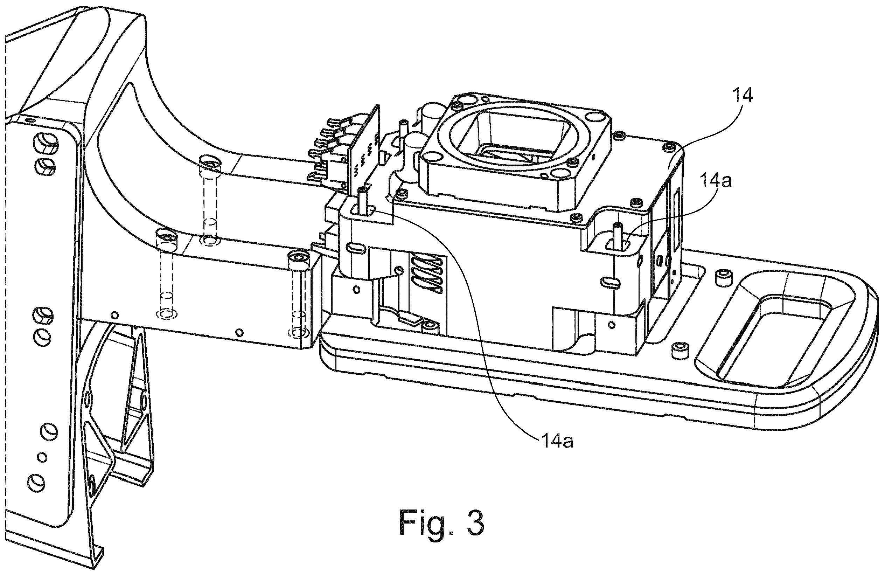

The heat exchanger plate has a main surface 3a, not visible but indicated by an arrow in Fig. 1a, which in operation serves for capturing heat emitted by an X-ray source and which may also be referred to as a bottom surface. Opposite of the main surface, the heat exchanger plate has a surface 3b, which may also be referred to as top surface of the heat exchanger plate. Moreover, the heat exchanger plate has outer sidewalls, collectively labelled 3c, and inner sidewalls, collectively labelled 3d. The inner sidewall are sidewalls delimiting one or more holes 7a, 7b in the heat exchanger plate. As an example, the hole 7a may be configured to accommodate a collimator 14, as explained in more detail below, e.g., making reference to Fig. 3.

The heat exchanger plate of this example also comprises grooves 8a and holes 8b, which may be used for assembly of the cooling system, e.g., by engaging with corresponding pins of a support arm, as will be explained in detail below. However, these holes and grooves are optional and additional or different elements for assembly may be provided depending on the assembly mechanism that allows for detachably attaching the TCU to the support arm.

A portion 9 comprising the elements for assembly is referred to as an interface section of TCU. It forms an interface with the support arm.

As can be seen in Fig. 1a, the top surface of the heat exchanger plate comprises optional holes 10. These are elements that may be used for attaching the heat exchanger plate to the monoblock of an X-ray source, as will be explained in more detail below.

As can be seen in Fig. 1a, the top surface of the heat exchanger plate comprises an optional recess 11 and the TCU comprises optional pins 12, which extend upwards from the top surface of the heat exchanger plate and may be attached to or formed integrally with the heat exchanger plate. These are elements that may be used for positioning a collimator on top of the TCU, as will be explained in more detail below.

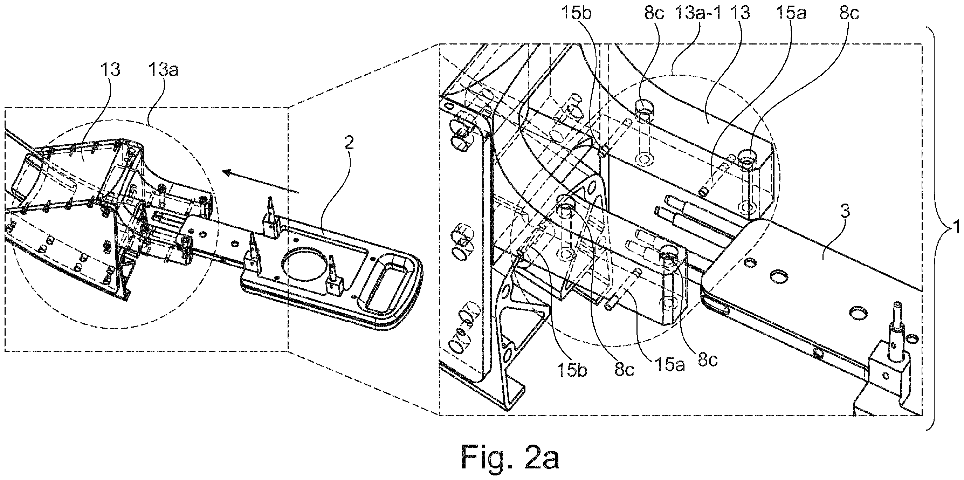

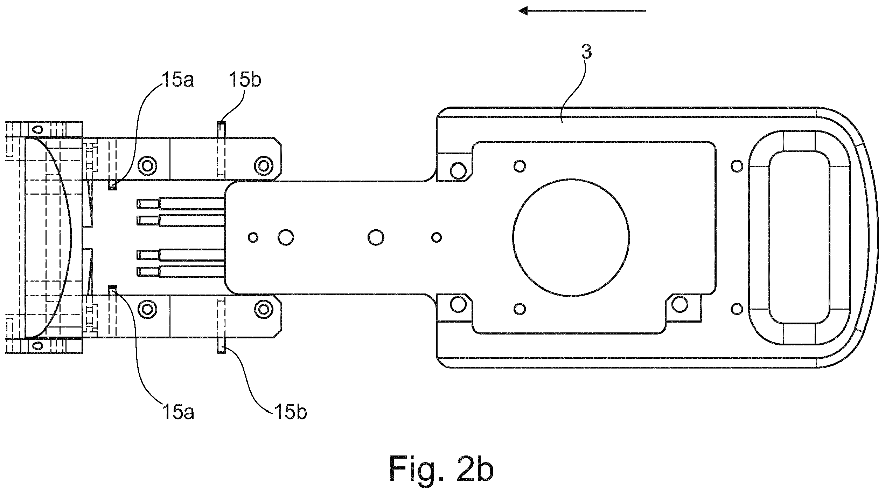

Figs. 2a and 2b illustrate, in an oblique view from the top and a top view, an assembly mechanism for detachably fixing a TCU according to the present disclosure, in particular a TCU as shown in Figs. 1a and 1b, to a support arm 13 of the cooling system 1 according to the present disclosure.

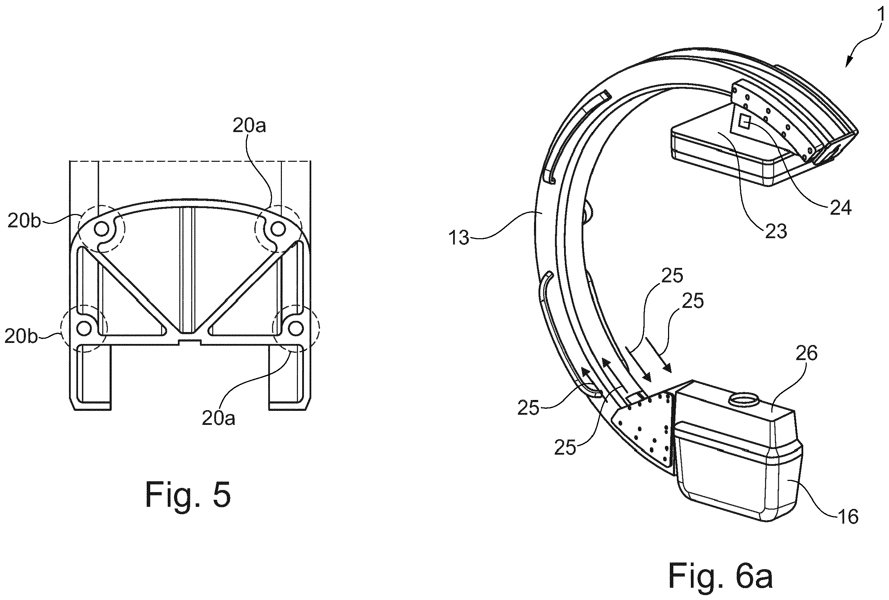

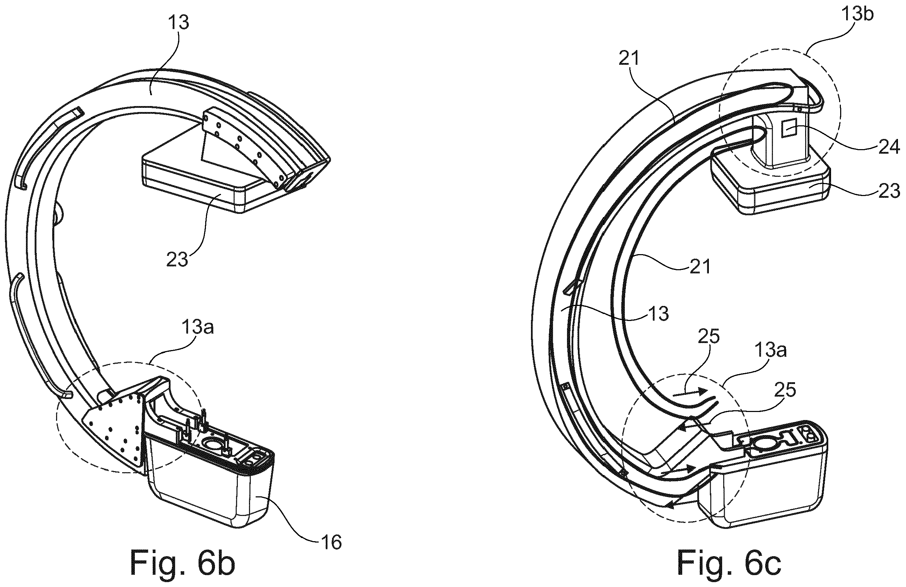

Figs. 2a and 2b show a first end 13a of the support arm. Not shown is the opposite end 13b of the support arm. Reference is made to Figs. 6a and 6b. The first end 13a comprises an interface section 13a-1 of the support arm.

The interface section 9 of the TCU and the interface section of the support arm are configured to engage with each other for detachably fixing the TCU to the support arm. In the present example, a first pair of pins 15a and a second pair of pins 15b are provided so as to engage with the grooves 8a and holes 8b of the heat exchanger plate, respectively. In the present example, the first pair of pins 15a are fixed, i.e., they are not independently movable. The second pair of pins are (independently) movable.

As an example, for assembly, the TCU may be shifted in the direction indicated by an arrow towards the interface section of the support arm such that the pins 15a are made to slide into the grooves 8a, thereby limiting movement of the TCU relative to the support arm.

Subsequently, the pins 15b may be inserted into the holes 8b, thereby fixing the position of the TCU relative to the support arm.

For detaching the TCU from the support arm, the pins 15b may be retracted from the holes 8b and the TCU may be slid in a direction opposite to the direction indicated by the arrow so as to disengage the grooves 8a from the pins 15a.

Thus, the interface section of the TCU and the interface section of the support arm are configured to provide an assembly mechanism configured such that the TCU is detachably fixed to the support arm in an assembled state.

However, additional and/or different elements providing a detachable assembly mechanism are conceivable.

Fig. 3 illustrates a TCU 2 and a portion of the support arm 13 of a cooling system according to the present disclosure in an assembled state. For illustration, the TCU and support arm as shown in Figs 1a, 1b, 2a, and 2b are shown, but other configurations are conceivable.

In addition, Fig. 3 illustrates a collimator 14 mounted over the TCU. It can be seen that the pins 12 of the TCU extend through holes 14a of the collimator 14, thereby positioning the collimator. A portion of the collimator is accommodated in the hole 7a in the heat exchanger plate (not visible in Fig. 3) and, if the TCU has the recess 11, the collimator may be arranged in said recess.

Thus, a mounting mechanism for the collimator may be provided, which may comprise the hole 7a, the recess 11, and the pins 12.

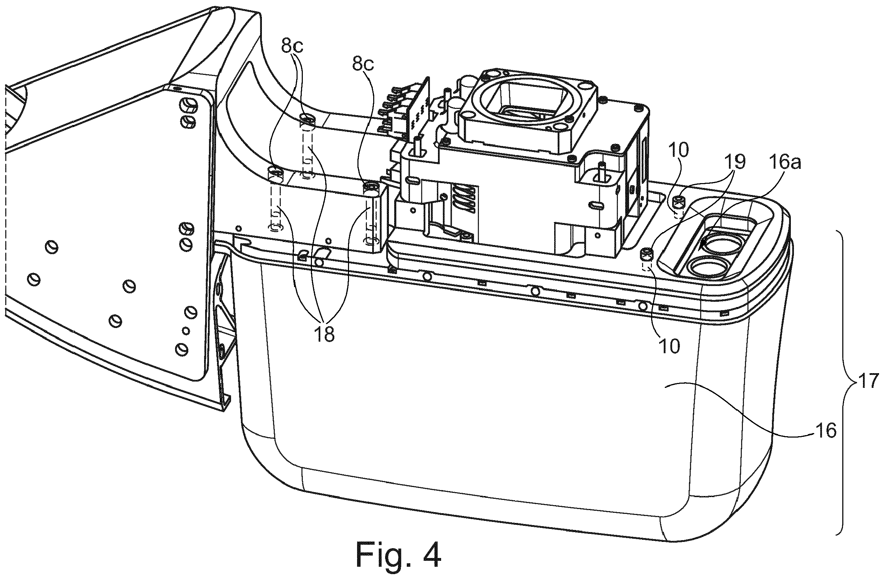

Fig. 4 illustrates schematically a TCU and a portion of the support arm of a cooling system according to the present disclosure, particularly as illustrated in Figs. 1a, 1b, 2a, 2b, and 3, and a monoblock 16 of an X-ray source 17, the monoblock mounted on the support arm.

In particular, Fig. 4 shows through holes 8c in the interface section of the support arm and screws 18 inserted in the through holes and used for mounting the monoblock on the support arm. As can be seen in Fig. 4, in an assembled state, the main surface of the heat exchanger plate is in contact with a top surface 16a of the monoblock. In operation, cooling fluid circulating through the heat exchanger plate may thus capture heat from the monoblock.

In this example, optional screws 19 extend through optional holes 10 of the heat exchanger plate so as to attach the heat exchanger plate to the monoblock of an X-ray source. Thus, the contact between the heat exchanger plate and the monoblock may be enforced.

Fig. 5 illustrates a cross section of an exemplary support arm 13 of the present disclosure, specifically, a cross section in a region that is outside of the interface section and does not form one of the ends of the support arm. In this example, the support arm is also the/an external heat exchanger to which the TCU may be connected. Specifically, in this example, the support arm comprises cooling channels 20a and 20b each having two ports (not shown), particularly an inlet port and an outlet port, at the interface section of the support arm. In an assembled state, the outlet ports of the support arm are connected with the inlet ports 5a and 5b of the TCU and the inlet ports of the support arm are connected to the outlet ports 6a and 6b of the TCU. For example, the ports of the support arm and the TCU may be configured and arranged such that they establish a plug connection between the TCU and the support arm.

Figs. 6a to 6c illustrate a fully assembled X-ray device 22 according to the present disclosure, the X-ray device comprising a TCU according to the present disclosure, for examples as illustrated in the preceding figures, and a support arm according to the present disclosure, for examples as illustrated in the preceding figures, particularly Fig. 5. The X-ray device in Figs. 6a to 6c further comprises a monoblock of an X-ray source, an X-ray detector 23 arranged at a second end 13b of the support arm, which is opposite to the first end 13a of the support arm, and a pump 24, which, in this example, is arranged at the second end 13b of the support arm, but may also be arranged at a different position. The pump is configured to circulate a cooling fluid through the support arm and the TCU.

In this example, the support arm is illustrated as a C-arc, but the support arm may also have a different shape. As implied by the lines 21 in Fig. 6c that illustrate an example of possible shapes of the cooling channels, the cooling channels of the support arm may extend along the entire length of the support arm.

Arrows 25 roughly indicate the fluid flow through the support arm.

Optionally, a detachable cover 26 may be provided covering the TCU and part of the collimator.

It is to be understood that the present disclosure is not limited to the support arm 13 as shown in Figs. 5, 6a, 6b, and 6c serving as an external heat exchanger connected to the TCU. That is, alternatively the ports of the TCU may be connected to different external heat exchanger.

While the invention has been illustrated and described in detail in the drawings and foregoing description, such illustration and description are to be considered exemplary and not restrictive. The invention is not limited to the disclosed embodiments. In view of the foregoing description and drawings it will be evident to a person skilled in the art that various modifications may be made within the scope of the invention, as defined by the claims.

### LIST OF REFERENCE SIGNS:

Cooling system 1
TCU 2
Heat exchanger plate 3
Main surface 3a
Top surface 3b
Outer sidewalls 3c
Inner sidewalls 3d
First end 3e
Second end 3f
Cooling fluid circuitry 4
Cooling fluid channels 4a, 4b,
Inlet port 5a, 5b
Outlet port 6a, 6b
Holes 7a, 7b
Grooves 8a
Holes 8b
Interface section 9
Holes 10
Recess 11
Pins 12
Support arm 13
First end 13a
Second end 13b
Interface section 13a-1
Collimator 14
Holes 14a
Pins 15a, 15b
Monoblock 16
X-ray source 17
Screws 18, 19
Cooling channels 20a, 20b
Lines 21
X-ray device 22
X-ray detector 23
Pump 24
Arrows 25
Detachable cover 26

## Claims

1. A cooling system (1) for an X-ray device (22), the cooling system (1) comprising
a top cooling unit, TCU, (2) comprising a heat exchanger plate (3) with an integrated cooling fluid circuitry (4); and
a support arm (13), particularly a C-arc, configured for detachably mounting the TCU (2) and for detachably mounting a monoblock (16) of an X-ray source (17) of the X-ray device,
wherein the fluid circuitry (4) is arranged over a main surface (3a) of the heat exchanger plate (3) so as to capture heat via the main surface (3a).

2. The cooling system according to claim 1,
wherein the integrated cooling fluid circuitry (4) comprises at least one, in particular two or more, integrated cooling fluid channels (4a, 4b), and
wherein each of the cooling fluid channels (4a, 4b) has two ports (5a, 5b, 6a, 6b), in particular, an inlet port (5a, 5b) and an outlet port (6a, 6b).

3. The cooling system (1) according to claim 1 or 2, wherein the TCU (2) comprises an interface section (9) configured to engage with an interface section (13a-1) of the support arm (13), in particular, wherein the interface section (9) of the TCU (2) and the interface section (13a-1) of the support arm (13) are configured to provide an assembly mechanism configured such that the TCU (2) is detachably fixed to the support arm (13) in an assembled state.

4. The cooling system (1) according to any of the preceding claims, wherein the support arm (13) comprises a mounting mechanism for detachably mounting the monoblock (16) to the support arm (13), in particular in such a manner that mounting and dismounting of the monoblock (16) does not require detaching the TCU (2) from the support arm (13).

5. The cooling system (1) of any of the preceding claims, the cooling system (1), in particular the TCU (2), comprising a mounting mechanism for detachably attaching the TCU (2) to the monoblock (16), in particular the top surface of the monoblock, mounted on the support arm (13).

6. The cooling system (1) according to any of the preceding claims, wherein the TCU (2) comprises a mounting mechanism for mounting a collimator (14) of the X-ray device (22), in particular, wherein the heat exchanger plate (3) has an opening in its main surface (3a) for accommodating at least a portion of the collimator (14) and optionally comprises positioning elements, e.g., pins, for positioning the collimator on top of the heat exchanger plate.

7. The cooling system (1) according to any of claims 2 to 6, wherein the support arm (13), in particular the C-arc, comprises one or more cooling channels (20a, 20b), each having two ports connected, in an assembled state, to the two ports (5a, 5b, 6a, 6b) of the or one of the cooling fluid channels (4a, 4b) of the cooling fluid circuitry (4), such that, in an assembled state, one or more closed cooling circuits are formed, each by at least one of the cooling channels (20a, 20b) of the support arm (13) and at least one of the cooling fluid channels (4a, 4b) of the heat exchanger plate (3).

8. The cooling system (1) according to any of claims 2 to 6, wherein the heat exchanger plate (3) and the support arm (13), in particular their respective interface sections, are configured such that the ports of the support arm (13) and the ports (5a, 5b, 6a, 6b) of the one or more cooling fluid channels (4a, 4b) are configured so as to form a plug connection with each other.

9. The cooling system (1) according to claim 8, comprising the/an assembly mechanism configured such that the TCU (2) is detachably fixed to the support arm (13), wherein the assembly mechanism comprises the plug connection and fixing elements, for example pins, configured to lock the relative position of the TCU (2) and the support arm (13) when the plug connection is in a connected state.

10. The cooling system (1) according to any of the preceding claims, further comprising one or more pumps (24) for circulating cooling fluid through the cooling fluid circuitry (4) of the TCU (2) and, in particular, through the closed cooling circuits formed by the cooling channels (20a, 20b) of the support arm and the cooling fluid channels (4a, 4b) of the heat exchanger plate (3).

11. The cooling system (1) according to any of the preceding claims, wherein the one or more integrated cooling fluid channels (4a, 4b) are arranged so as to optimize heat distribution throughout the heat exchanger plate (3).

12. The cooling system according to any of the previous claims, wherein the TCU (2) is arranged for mounting a collimator (14) of the X-ray device (22) such that the heat exchange plate (3) is located between the monoblock (16) and the collimator (14), and wherein the integrated cooling fluid circuitry (4) of the heat exchange plate (3) comprises a portion of channel (4b) extending beneath the collimator (14) once mounted and above the monoblock (16), and in particular wherein the heat exchanger plate (3) has an opening in its main surface (3a) for accommodating at least a portion of the collimator (14) and a shape of said portion of channel (4b) closely follows an external shape of this opening.

13. The cooling system according to any of the preceding claims, wherein the cooling channels (20a, 20b) of the support arm (13) extend along the entire length of the support arm (13).

14. An X-ray device (22) comprising the cooling system (1) according to any of the preceding claims and an X-ray source (17) comprising a monoblock (16), wherein, in an assembled state, the main surface (3a) of the heat exchanger plate (3) is in contact with the top surface of the monoblock (16).

15. The X-ray device (22) according to claim 14, further comprising an X-ray detector (23) at the end of the support arm (13) opposite to the end of the support arm on which the monoblock (16) and TCU (2) are mounted.
